# EUROPEAN PATENT APPLICATION

(11) **EP 1 304 118 A2**
(43) Date of publication of application: **23.04.2003**
(21) Application number: 02028360.2
(22) Date of filing: 31.10.1997
(51) Int. Cl.: A61K 38/17, A61K 9/12, A61P 11/00, A61P 31/00

(54) **Therapeutic uses and formulations for BPI protein products.**

(30) Priority: 01.11.1996 US 742986
(62) Divisional of application: 97946427.8
(71) Applicant: XOMA Technology Ltd., Berkeley, CA 94710 (US)
(72) Inventor: Carroll, Stephen, Fitzhugh, Valnut Creek, CA 94596 (US); Scannon, Patrick, J., San Francisco, CA 94117 (US); Gavit, Patrick, D., Covina, CA 91722 (US)
(74) Representative: Richardson, Kate

(57) **Abstract**

Improved therapeutic uses and formulations of BPI protein products for aerosol delivery are described.

SEQUENCE LISTING

(1) GENERAL INFORMATION:

(i) APPLICANT: XOMA Corporation

(ii) TITLE OF INVENTION: IMPROVED THERAPEUTIC USES OF BPI PROTEIN
PRODUCTS IN CYSTIC FIBROSIS PATIENTS

(iii) NUMBER OF SEQUENCES: 2

(iv) CORRESPONDENCE ADDRESS:
(A) ADDRESSEE: Marshall, O'Toole, Gerstein, Murray & Borun
(B) STREET: 6300 Sears Tower, 233 South Wacker Drive
(C) CITY: Chicago
(D) STATE: Illinois
(E) COUNTRY: United States of America
(F) ZIP: 60606-6402

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: Patent In Release #1.0, Version #1.25

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: 08/742,986
(B) FILING DATE: 1-NOV-1996
(C) CLASSIFICATION:

(viii) ATTORNEY/AGENT INFORMATION:
(A) NAME: Rin-Laures, Li-Hsien
(B) REGISTRATION NUMBER: 33,547
(C) REFERENCE/DOCKET NUMBER: 27129/34309

(ix) TELECOMMUNICATION INFORMATION:
(A) TELEPHONE: 312/474-6300
(B) TELEFAX: 312/474-0448
(C) TELEX: 25-3856

(2) INFORMATION FOR SEQ ID NO:1:

(i) SEQUENCE CHARACTERISTICS:
(A) LENGTH: 1813 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 31..1491

(ix) FEATURE:
(A) NAME/KEY: mat_peptide
(B) LOCATION: 124..1491

(ix) FEATURE:
(A) NAME/KEY: misc_feature

(D) OTHER INFORMATION: "rBPI"

(xi) SEQUENCE DESCRIPTION: SEQ ID NO:1:

CAGGCCTTGA GGTTTTGGCA GCTCTGGAGG ATG AGA GAG AAC ATG GCC AGG GGC 54
Met Arg Glu Asn Met Ala Arg Gly
-31 -30 -25

CCT TGC AAC GCG CCG AGA TGG GTG TCC CTG ATG GTG CTC GTC GCC ATA 102
Pro Cys Asn Ala Pro Arg Trp Val Ser Leu Met Val Leu Val Ala Ile
-20 -15 -10

GGC ACC GCC GTG ACA GCG GCC GTC AAC CCT GGC GTC GTG GTC AGG ATC 150
Gly Thr Ala Val Thr Ala Ala Val Asn Pro Gly Val Val Val Arg Ile
-5 1 5

TCC CAG AAG GGC CTG GAC TAC GCC AGC CAG CAG GGG ACG GCC GCT CTG 198
Ser Gln Lys Gly Leu Asp Tyr Ala Ser Gln Gln Gly Thr Ala Ala Leu
10 15 20 25

CAG AAG GAG CTG AAG AGG ATC AAG ATT CCT GAC TAC TCA GAC AGC TTT 246
Gln Lys Glu Leu Lys Arg Ile Lys Ile Pro Asp Tyr Ser Asp Ser Phe
30 35 40

AAG ATC AAG CAT CTT GGG AAG GGG CAT TAT AGC TTC TAC AGC ATG GAC 294
Lys Ile Lys His Leu Gly Lys Gly His Tyr Ser Phe Tyr Ser Met Asp
45 50 55

ATC CGT GAA TTC CAG CTT CCC AGT TCC CAG ATA AGC ATG GTG CCC AAT 342
Ile Arg Glu Phe Gln Leu Pro Ser Ser Gln Ile Ser Met Val Pro Asn
60 65 70

GTG GGC CTT AAG TTC TCC ATC AGC AAC GCC AAT ATC AAG ATC AGC GGG 390
Val Gly Leu Lys Phe Ser Ile Ser Asn Ala Asn Ile Lys Ile Ser Gly
75 80 85

AAA TGG AAG GCA CAA AAG AGA TTC TTA AAA ATG AGC GGC AAT TTT GAC 438
Lys Trp Lys Ala Gln Lys Arg Phe Leu Lys Met Ser Gly Asn Phe Asp
90 95 100 105

CTG AGC ATA GAA GGC ATG TCC ATT TCG GCT GAT CTG AAG CTG GGC AGT 486
Leu Ser Ile Glu Gly Met Ser Ile Ser Ala Asp Leu Lys Leu Gly Ser
110 115 120

AAC CCC ACG TCA GGC AAG CCC ACC ATC ACC TGC TCC AGC TGC AGC AGC 534
Asn Pro Thr Ser Gly Lys Pro Thr Ile Thr Cys Ser Ser Cys Ser Ser
125 130 135

CAC ATC AAC AGT GTC CAC GTG CAC ATC TCA AAG AGC AAA GTC GGG TGG 582
His Ile Asn Ser Val His Val His Ile Ser Lys Ser Lys Val Gly Trp
140 145 150

CTG ATC CAA CTC TTC CAC AAA AAA ATT GAG TCT GCG CTT CGA AAC AAG 630

Leu Ile Gln Leu Phe His Lys Lys Ile Glu Ser Ala Leu Arg Asn Lys
    155                 160                 165

ATG AAC AGC CAG GTC TGC GAG AAA GTG ACC AAT TCT GTA TCC TCC AAG     678
Met Asn Ser Gln Val Cys Glu Lys Val Thr Asn Ser Val Ser Ser Lys
170                 175                 180                 185

CTG CAA CCT TAT TTC CAG ACT CTG CCA GTA ATG ACC AAA ATA GAT TCT     726
Leu Gln Pro Tyr Phe Gln Thr Leu Pro Val Met Thr Lys Ile Asp Ser
                190                 195                 200

GTG GCT GGA ATC AAC TAT GGT CTG GTG GCA CCT CCA GCA ACC ACG GCT     774
Val Ala Gly Ile Asn Tyr Gly Leu Val Ala Pro Pro Ala Thr Thr Ala
            205                 210                 215

GAG ACC CTG GAT GTA CAG ATG AAG GGG GAG TTT TAC AGT GAG AAC CAC     822
Glu Thr Leu Asp Val Gln Met Lys Gly Glu Phe Tyr Ser Glu Asn His
        220                 225                 230

CAC AAT CCA CCT CCC TTT GCT CCA CCA GTG ATG GAG TTT CCC GCT GCC     870
His Asn Pro Pro Pro Phe Ala Pro Pro Val Met Glu Phe Pro Ala Ala
    235                 240                 245

CAT GAC CGC ATG GTA TAC CTG GGC CTC TCA GAC TAC TTC TTC AAC ACA     918
His Asp Arg Met Val Tyr Leu Gly Leu Ser Asp Tyr Phe Phe Asn Thr
250                 255                 260                 265

GCC GGG CTT GTA TAC CAA GAG GCT GGG GTC TTG AAG ATG ACC CTT AGA     966
Ala Gly Leu Val Tyr Gln Glu Ala Gly Val Leu Lys Met Thr Leu Arg
                270                 275                 280

GAT GAC ATG ATT CCA AAG GAG TCC AAA TTT CGA CTG ACA ACC AAG TTC     1014
Asp Asp Met Ile Pro Lys Glu Ser Lys Phe Arg Leu Thr Thr Lys Phe
            285                 290                 295

TTT GGA ACC TTC CTA CCT GAG GTG GCC AAG AAG TTT CCC AAC ATG AAG     1062
Phe Gly Thr Phe Leu Pro Glu Val Ala Lys Lys Phe Pro Asn Met Lys
        300                 305                 310

ATA CAG ATC CAT GTC TCA GCC TCC ACC CCG CCA CAC CTG TCT GTG CAG     1110
Ile Gln Ile His Val Ser Ala Ser Thr Pro Pro His Leu Ser Val Gln
    315                 320                 325

CCC ACC GGC CTT ACC TTC TAC CCT GCC GTG GAT GTC CAG GCC TTT GCC     1158
Pro Thr Gly Leu Thr Phe Tyr Pro Ala Val Asp Val Gln Ala Phe Ala
330                 335                 340                 345

GTC CTC CCC AAC TCC TCC CTG GCT TCC CTC TTC CTG ATT GGC ATG CAC     1206
Val Leu Pro Asn Ser Ser Leu Ala Ser Leu Phe Leu Ile Gly Met His
                350                 355                 360

ACA ACT GGT TCC ATG GAG GTC AGC GCC GAG TCC AAC AGG CTT GTT GGA     1254
Thr Thr Gly Ser Met Glu Val Ser Ala Glu Ser Asn Arg Leu Val Gly
            365                 370                 375

GAG CTC AAG CTG GAT AGG CTG CTC CTG GAA CTG AAG CAC TCA AAT ATT     1302
Glu Leu Lys Leu Asp Arg Leu Leu Leu Glu Leu Lys His Ser Asn Ile
        380                 385                 390

GGC CCC TTC CCG GTT GAA TTG CTG CAG GAT ATC ATG AAC TAC ATT GTA     1350
Gly Pro Phe Pro Val Glu Leu Leu Gln Asp Ile Met Asn Tyr Ile Val
    395                 400                 405

CCC ATT CTT GTG CTG CCC AGG GTT AAC GAG AAA CTA CAG AAA GGC TTC 1398
Pro Ile Leu Val Leu Pro Arg Val Asn Glu Lys Leu Gln Lys Gly Phe
410 415 420 425

CCT CTC CCG ACG CCG GCC AGA GTC CAG CTC TAC AAC GTA GTG CTT CAG 1446
Pro Leu Pro Thr Pro Ala Arg Val Gln Leu Tyr Asn Val Val Leu Gln
430 435 440

CCT CAC CAG AAC TTC CTG CTG TTC GGT GCA GAC GTT GTC TAT AAA 1491
Pro His Gln Asn Phe Leu Leu Phe Gly Ala Asp Val Val Tyr Lys
445 450 455

TGAAGGCACC AGGGGTGCCG GGGGCTGTCA GCCGCACCTG TTCCTGATGG GCTGTGGGGC 1551

ACCGGCTGCC TTTCCCCAGG GAATCCTCTC CAGATCTTAA CCAAGAGCCC CTTGCAAACT 1611

TCTTCGACTC AGATTCAGAA ATGATCTAAA CACGAGGAAA CATTATTCAT TGGAAAAGTG 1671

CATGGTGTGT ATTTTAGGGA TTATGAGCTT CTTTCAAGGG CTAAGGCTGC AGAGATATTT 1731

CCTCCAGGAA TCGTGTTTCA ATTGTAACCA AGAAATTTCC ATTTGTGCTT CATGAAAAAA 1791

AACTTCTGGT TTTTTTCATG TG 1813

(2) INFORMATION FOR SEQ ID NO:2:

(i) SEQUENCE CHARACTERISTICS:
(A) LENGTH: 487 amino acids
(B) TYPE: amino acid
(D) TOPOLOGY: linear

(ii) MOLECULE TYPE: protein

(xi) SEQUENCE DESCRIPTION: SEQ ID NO:2:

Met Arg Glu Asn Met Ala Arg Gly Pro Cys Asn Ala Pro Arg Trp Val
-31 -30 -25 -20

Ser Leu Met Val Leu Val Ala Ile Gly Thr Ala Val Thr Ala Ala Val
-15 -10 -5 1

Asn Pro Gly Val Val Val Arg Ile Ser Gln Lys Gly Leu Asp Tyr Ala
                5                   10                  15

Ser Gln Gln Gly Thr Ala Ala Leu Gln Lys Glu Leu Lys Arg Ile Lys
            20                  25                  30

Ile Pro Asp Tyr Ser Asp Ser Phe Lys Ile Lys His Leu Gly Lys Gly
        35                  40                  45

His Tyr Ser Phe Tyr Ser Met Asp Ile Arg Glu Phe Gln Leu Pro Ser
50 55 60 65

Ser Gln Ile Ser Met Val Pro Asn Val Gly Leu Lys Phe Ser Ile Ser
70 75 80

Asn Ala Asn Ile Lys Ile Ser Gly Lys Trp Lys Ala Gln Lys Arg Phe
                85                  90                  95

Leu Lys Met Ser Gly Asn Phe Asp Leu Ser Ile Glu Gly Met Ser Ile
            100                 105                 110

Ser Ala Asp Leu Lys Leu Gly Ser Asn Pro Thr Ser Gly Lys Pro Thr
        115                 120                 125

Ile Thr Cys Ser Ser Cys Ser Ser His Ile Asn Ser Val His Val His
130 135 140 145

Ile Ser Lys Ser Lys Val Gly Trp Leu Ile Gln Leu Phe His Lys Lys
150 155 160

Ile Glu Ser Ala Leu Arg Asn Lys Met Asn Ser Gln Val Cys Glu Lys
                165                 170                 175

Val Thr Asn Ser Val Ser Ser Lys Leu Gln Pro Tyr Phe Gln Thr Leu
            180                 185                 190

Pro Val Met Thr Lys Ile Asp Ser Val Ala Gly Ile Asn Tyr Gly Leu
        195                 200                 205

Val Ala Pro Pro Ala Thr Thr Ala Glu Thr Leu Asp Val Gln Met Lys
210 215 220 225

Gly Glu Phe Tyr Ser Glu Asn His His Asn Pro Pro Pro Phe Ala Pro
230 235 240

Pro Val Met Glu Phe Pro Ala Ala His Asp Arg Met Val Tyr Leu Gly
                245                 250                 255

Leu Ser Asp Tyr Phe Phe Asn Thr Ala Gly Leu Val Tyr Gln Glu Ala
            260                 265                 270

Gly Val Leu Lys Met Thr Leu Arg Asp Asp Met Ile Pro Lys Glu Ser
        275                 280                 285

Lys Phe Arg Leu Thr Thr Lys Phe Phe Gly Thr Phe Leu Pro Glu Val
290 295 300 305

Ala Lys Lys Phe Pro Asn Met Lys Ile Gln Ile His Val Ser Ala Ser
310 315 320

Thr Pro Pro His Leu Ser Val Gln Pro Thr Gly Leu Thr Phe Tyr Pro
                325                 330                 335

Ala Val Asp Val Gln Ala Phe Ala Val Leu Pro Asn Ser Ser Leu Ala
            340                 345                 350

Ser Leu Phe Leu Ile Gly Met His Thr Thr Gly Ser Met Glu Val Ser
        355                 360                 365

Ala Glu Ser Asn Arg Leu Val Gly Glu Leu Lys Leu Asp Arg Leu Leu
370 375 380 385

Leu Glu Leu Lys His Ser Asn Ile Gly Pro Phe Pro Val Glu Leu Leu
390 395 400

Gln Asp Ile Met Asn Tyr Ile Val Pro Ile Leu Val Leu Pro Arg Val
                405                 410                 415

Asn Glu Lys Leu Gln Lys Gly Phe Pro Leu Pro Thr Pro Ala Arg Val
            420                 425                 430

Gln Leu Tyr Asn Val Val Leu Gln Pro His Gln Asn Phe Leu Leu Phe
        435                 440                 445

Gly Ala Asp Val Val Tyr Lys
450                 455

## Description

### BACKGROUND OF THE INVENTION

The present invention relates generally to novel improved methods of treating cystic fibrosis patients by administering N-terminal bactericidal/permeability-increasing protein (BPI) protein products. The present invention also relates generally to improved formulations for aerosol delivery to cystic fibrosis patients of BPI protein products alone or in combination with other therapeutic agents.

Cystic fibrosis (CF) is the most common lethal inherited disorder among Caucasian populations, affecting between 1 in 2000 to 1 in 4500 children. CF is a recessive disorder resulting from a defect in the cystic fibrosis transmembrane conductance regulator (CFTR) gene, a member of the ATP binding cassette (ABC) superfamily, located on the long arm of chromosome seven, that is thought to encode a cAMP-regulated chloride ion channel. CF is characterized by chronic pulmonary infection and colonization of the lungs by gram-negative bacteria (predominantly *Pseudomonas aeruginosa*), pulmonary inflammation, and progressive pulmonary damage, as well as pancreatic insufficiency. There is prominent pulmonary neutrophil infiltration, and levels of the neutrophil enzyme elastase found in the sputum of CF patients are so high as to overwhelm the host's elastase inhibitor α₁-antitrypsin. In addition, CF is associated with various extra-pulmonary autoimmune phenomena, including arthropathy, liver disease resembling sclerosing cholangitis, and both cutaneous and systemic vasculitis. Due to improvements in therapy, more than 25 % of the patients reach adulthood and more than 9% live past the age of 30. [Harrison's *Principles of Internal Medicine,* 13th ed., Isselbacher et al., eds., McGraw-Hill, NY.]

Pulmonary treatment of cystic fibrosis patients requires delivery of therapeutic quantities of drug to the lungs. This is typically done by inhaling either an aerosol or dry powder form of the drug. Aerosol delivery of proteins can be accomplished using either a nebulizer or a metered dose inhaler. There are two basic types of nebulizers: jet and ultrasonic.

Jet nebulizers make use of the Bernoulli principle; a stream of air or oxygen from compressed cylinder or compressor is passed through a narrow constriction known as a venturi, thereby generating an area of low pressure which causes drug solution from a reservoir to be drawn up into the venturi, where it is fragmented into droplets by the airstream. Only the smallest droplets exit the nebulizer while the others impact on a baffle and return to the reservoir. Droplet size for jet nebulizers is inversely proportional to the air flow rate. Ultrasonic nebulizers use a rapidly vibrating piezoelectric crystal to create small droplets. Ultrasonic vibrations from the crystal produce standing waves on the surface of the drug solution. Droplets then break free from the wave crests. Droplet size for ultrasonic nebulizers is inversely proportional to the ultrasonic frequency. Jet nebulizers tend to produce smaller droplets and cause less cough and irritation than ultrasonic nebulizers.

The lung deposition characteristics and efficacy of an aerosol depend largely on the particle or droplet size. Generally, the smaller the droplet, the greater its chance of peripheral penetration and retention. Very fine particles below 0.5 µm in diamater, however, may be exhaled without being deposited. One study reported that central airway deposition peaks at 6-7 µm and peripheral airway deposition at 2-3 µm. Particles with a diameter in the range of about 1 to about 5 µm are thus generally accepted as the target droplet size for delivery of pharmaceutical aerosols [O'Callaghan et al., *Thorax*, *52*:531-544 (1997)], while droplet sizes in the range of about 1 to about 3 µm are useful for reaching the alveolar portion of the lung.

The efficiency of drug delivery to the lungs depends on a variety of factors, including nebulizer type, airflow rate, drug formulation components, drug concentration and drug volume. Formulation components may also affect the incidence of adverse side effects such as throat irritation, coughing and bronchoconstriction. For example, osmolality affects bronchoconstriction. [Fine et al., *Am. Rev. Respir. Dis., 135*:826-830 (1987); Balmes et al., *Am. Rev. Respir. Dis., 138*:35-39 (1988).] Certain buffer salts can lead to irritation of the throat and coughing. [Godden et al., *Clinical Sci., 70*:301-306 (1986); Auffarth et al., *Thorax, 46*:638-642 (1991); Snell, *Respir. Med., 84*:345-348 (1990).] In one study, solutions of urea, water, sodium acetate and sodium bicarbonate increased coughing while a solution of sodium chloride did not. [Godden et al., *supra*.] In addition, for non-isotonic solutions, uptake or loss of water in the airways can change droplet size distribution; for this reason, formulations are generally recommended to be isotonic. [Gonda et al., in *Particle Size Analysis*, Stanley-Wood, ed., Wiley Heyden Ltd., New York, NY (1983), page 52; Gonda et al., in *Aerosols,* Masuda and Takahashi, eds., Pergamon Press, New York, NY (1991), pages 227-230.] Formulations having a pH of 5.0 or greater are reported to minimize side effects. [Beasley et al., *Br. J. Clin. Pharmacol*., *25*:283-287 (1988).]

Delivery efficiency DE (defined as the percentage of drug in the nebulizer which reaches the lung) is the product of nebulizer efficiency NE (percentage of drug which exits the nebulizer) and respirable fraction RF (percentage of aerosol droplets which have exited the nebulizer that are of the correct size range for deposition in the lungs). The following equation summarizes the relationship: DE = NE x RF.

The nebulization process can be very harsh for proteins because it increases the exposure of protein molecules to the air-liquid interface, which results in some cases in denaturation and subsequent precipitation of the protein. Therefore, a need exists for improved formulations which can be delivered by nebulization with good nebulizer efficiency and delivery efficiency.

Anti-neutrophil cytoplasmic antibodies (ANCA) have been recognized as a class of autoantibodies that react with the endogenous cytoplasmic constituents of neutrophils and monocytes. ANCA are detected by indirect immunofluorescence (IIF) on ethanol-fixed neutrophils. The presence of ANCA has been associated with some cystic fibrosis patients, with various idiopathic systemic vasculitis disorders (*i.e*., inflammation of and damage to the blood vessels) and with other inflammatory disorders, and can be diagnostic of certain vasculitides. These vasculitides are sometimes called ANCA-associated vasculitides (AAV). A pathophysiologic role for ANCA in vasculitides has been proposed but remains to be definitively established. [Kallenberg et al., *Clin. Exp. Immunol., 100*:1-3 (1995).] Some of the antigens recognized by ANCA have been identified, such as proteinase-3 (PR-3) and myeloperoxidase (MPO).

Zhao et al., *Q*.*J*.*M*., *89(4)*:259-265 (1996) report that sera from 60/66 (91%) adult CF patients had autoantibodies to BPI. The specificity of these antibodies was confirmed by inhibition studies with purified BPI. None of these 66 samples recognized PR-3 or MPO, and only 21 (32%) of the 66 samples were cANCA-positive by IIF. Thus, BPI was identified as the major ANCA antigen in CF. Furthermore, the levels of anti-BPI antibody, particularly anti-BPI IgA, significantly correlated with clinical parameters such as reductions in pulmonary function and the presence of secondary vasculitis. Zhao et al. suggested that the late autoimmune complications observed in CF patients might be related to anti-BPI autoantibodies, which may also be involved in the activation of neutrophils and tissue damage in the lungs.

BPI is a protein isolated from the granules of mammalian polymorphonuclear leukocytes (PMNs or neutrophils), which are blood cells essential in the defense against invading microorganisms. Human BPI protein has been isolated from PMNs by acid extraction combined with either ion exchange chromatography [Elsbach, *J. Biol. Chem*., *254*:11000 (1979)] or *E. coli* affinity chromatography [Weiss, et al., *Blood, 69:652* (1987)]. BPI obtained in such a manner is referred to herein as natural BPI and has been shown to have potent bactericidal activity against a broad spectrum of gram-negative bacteria. The molecular weight of human BPI is approximately 55,000 daltons (55 kD). The amino acid sequence of the entire human BPI protein and the nucleic acid sequence of DNA encoding the protein have been reported in Figure 1 of Gray et al., *J. Biol. Chem., 264*:9505 (1989), incorporated herein by reference. The Gray et al. amino acid sequence is set out in SEQ ID NO: 1 hereto. U.S. Patent No. 5,198,541 discloses recombinant genes encoding and methods for expression of BPI proteins, including BPI holoprotein and fragments of BPI.

BPI is a strongly cationic protein. The N-terminal half of BPI accounts for the high net positive charge; the C-terminal half of the molecule has a net charge of -3. [Elsbach and Weiss (1981), *supra*.] *A* proteolytic N-terminal fragment of BPI having a molecular weight of about 25 kD possesses essentially all the anti-bacterial efficacy of the naturally-derived 55 kD human BPI holoprotein. [Ooi et al., *J. Bio. Chem*., *262:* 14891-14894 (1987)]. In contrast to the N-terminal portion, the C-terminal region of the isolated human BPI protein displays only slightly detectable anti-bacterial activity against gram-negative organisms. [Ooi et al., *J. Exp. Med., 174*:649 (1991).] An N-terminal BPI fragment of approximately 23 kD, referred to as "rBPI₂₃," has been produced by recombinant means and also retains anti-bacterial activity against gram-negative organisms. [Gazzano-Santoro et al., *Infect. Immun. 60*:4754-4761 (1992).] An N-terminal analog of BPI, rBPI₂₁, has been produced as described in Horwitz et al., *Protein Expression Purification, 8*:28-40 (1996).

The bactericidal effect of BPI has been reported to be highly specific to gram-negative species, e.g., in Elsbach and Weiss, *Inflammation: Basic Principles and Clinical Correlates,* eds. Gallin et al., Chapter 30, Raven Press, Ltd. (1992). The precise mechanism by which BPI kills gram-negative bacteria is not yet completely elucidated, but it is believed that BPI must first bind to the surface of the bacteria through electrostatic and hydrophobic interactions between the cationic BPI protein and negatively charged sites on LPS. In susceptible gram-negative bacteria, BPI binding is thought to disrupt LPS structure, leading to activation of bacterial enzymes that degrade phospholipids and peptidoglycans, altering the permeability of the cell's outer membrane, and initiating events that ultimately lead to cell death. [Elsbach and Weiss (1992), *supra*]. LPS has been referred to as "endotoxin" because of the potent inflammatory response that it stimulates, i.e., the release of mediators by host inflammatory cells which may ultimately result in irreversible endotoxic shock. BPI binds to lipid A, reported to be the most toxic and most biologically active component of LPS.

BPI protein products, as discussed *infra*, have a wide variety of beneficial activities in addition to their gram-negative bactericidal activities. The observation of antibodies that are reactive against BPI among cystic fibrosis patients suggests that these antibodies may interfere with the activities of BPI. A need therefore exists for improved methods of treating cystic fibrosis patients that have BPI-reactive antibodies with BPI protein products.

### SUMMARY OF THE INVENTION

The present invention provides novel improved methods of treating cystic fibrosis patients that have non N-terminal-BPI-reactive antibodies by administering N-terminal bactericidal/permeability-increasing (BPI) protein products. The invention is based on the discovery that BPI-reactive antibodies in cystic fibrosis patients bind to BPI holoprotein but have little or no reactivity with N-terminal BPI protein products. Interference with the beneficial activities of endogenous BPI or exogenous BPI protein products can therefore be avoided by administering N-terminal BPI protein products.

It is contemplated that these improved methods will be useful when the N-terminal BPI protein product is being administered for any of the indications presently known for BPI protein products. For example, the N-terminal BPI protein product may be administered to a human subject to ameliorate adverse effects associated with endotoxin in circulation, meningococcemia, hemorrhagic trauma, burn trauma, ischemia/reperfusion injury, or liver resection injury. A N-terminal BPI protein product may also be administered for the treatment of gram-negative bacterial infection, gram-positive bacterial or mycoplasmal infection, fungal infection, protozoal infection, chlamydial infection, mycobacterial infection, chronic inflammatory diseases, including rheumatoid and reactive arthritis, or to enhance the effectiveness of antimicrobial activity, or to inhibit angiogenesis or to promote fibrinolysis.

Presently preferred N-terminal BPI protein products include amino-terminal fragments of BPI having a molecular weight of about 20 kD to 25 kD, rBPI₂₃ or a dimeric form thereof, and rBPI₂₁.

It is contemplated that the administration of BPI protein products, especially N-terminal BPI protein products, according to all aspects of the present invention may be accompanied by the concurrent administration of other therapeutic agents such as antimicrobial agents, including antibiotics and anti-fungal agents, or agents such as DNAase (Pulmozyme®).

The invention also contemplates compositions for aerosol delivery comprising a BPI protein product and a poloxamer (polyoxypropylene-polyoxyethylene block copolymer) surfactant at a concentration of 0.3% or more. Such compositions are useful in methods for treating cystic fibrosis patients with BPI protein products.

Numerous additional aspects and advantages of the invention will become apparent to those skilled in the art upon consideration of the following detailed description of the invention which describes presently preferred embodiments thereof.

### DETAILED DESCRIPTION OF THE INVENTION

The present invention provides improved methods of treating cystic fibrosis patients that have non-N-terminal-BPI-reactive antibodies, the presence of which may interfere with the activities of BPI protein products in these subjects, by the administration of N-terminal BPI protein products. The invention is based on the discovery that BPI-reactive autoantibodies in cystic fibrosis patients bind to BPI holoprotein but have little or no reactivity with N-terminal BPI protein products; the ANCA-recognized epitopes thus appear to reside predominantly outside the N-terminal 193 amino acids of BPI.

BPI protein products are known to have a variety of beneficial activities. BPI protein products are known to be bactericidal for gram-negative bacteria, as described in U.S. Patent Nos. 5,198,541 and 5,523,288, both of which are incorporated herein by reference. BPI protein products are also known to enhance the effectiveness of antibiotic therapy in gram-negative bacterial infections, as described in U.S. Patent No. 5,523,288, which is incorporated herein by reference. BPI protein products are also known to be bactericidal for gram-positive bacteria and mycoplasma, and to enhance the effectiveness of antibiotics in gram-positive bacterial infections, as described in co-owned, co-pending U.S. Application Serial No. 08/372,783 filed January 13, 1995, which is in turn a continuation-in-part of U.S. Application Serial No. 08/274,299 filed July 11, 1994, and corresponding International Publication No. WO 95/08344 (PCT/US94/11225), all of which are incorporated herein by reference. BPI protein products are further known to exhibit anti-fungal activity, and to enhance the activity of other anti-fungal agents, as described in co-owned, co-pending U.S. Application Serial No. 08/372,105 filed January 13, 1995, which is in turn a continuation-in-part of U.S. Application Serial No. 08/273,540 filed July 11, 1994, and corresponding International Publication No. WO 95/19179 (PCT/US95/00498), and further as described for anti-fungal peptides in co-owned, co-pending U.S. Application Serial No. 08/621,259 filed March 21, 1996, which is in turn a continuation-in-part of U.S. Application Serial No. 08/504,841 filed July 20, 1994 and corresponding International Publication No. WO 96/08509 (PCT/US95/09262) and PCT Application No. PCT/US96/03845, all of which are incorporated herein by reference. BPI protein products are further known to exhibit anti-protozoan activity, as described in co-owned, co-pending U.S. Application Serial No. 08/273,470 filed July 11, 1994 and corresponding International Publication No. WO 96/01647 (PCT/US95/08624), all of which are incorporated herein by reference. BPI protein products are known to exhibit anti-chlamydial activity, as described in co-owned, co-pending U.S. Application Serial No. 08/694,843 filed August 9, 1996, all of which are incorporated herein by reference. Finally, BPI protein products are known to exhibit anti-mycobacterial activity, as described in co-owned, co-pending U.S. Application Serial No. 08/626,646 filed April 1, 1996, which is in turn a continuation of U.S. Application Serial No. 08/285,803 filed August 14, 1994, which is in turn a continuation-in-part of U.S. Application Serial No. 08/031,145 filed March 12, 1993 and corresponding International Publication No. WO94/20129 (PCT/US94/02463), all of which are incorporated herein by reference.

The effects of BPI protein products in humans with endotoxin in circulation, including effects on TNF, IL-6 and endotoxin are described in co-owned, co-pending U.S. Application Serial No. 08/378,228, filed January 24, 1995, which in turn is a continuation-in-part application of U.S. Serial No. 08/291,112, filed August 16, 1994, which in turn is a continuation-in-part application of U.S. Serial No. 08/188,221, filed January 24, 1994, and corresponding International Publication No. WO 95/19784 (PCT/US95/01151), all of which are incorporated herein by reference.

BPI protein products are also known to be useful for treatment of specific disease conditions, such as meningococcemia in humans (as described in co-owned, co-pending U.S. Application Serial No. 08/644,287 filed May 10, 1996, incorporated herein by reference), hemorrhagic trauma in humans, (as described in co-owned, co-pending U.S. Application Serial No. 08/652,292 filed May 23, 1996, incorporated herein by reference), burn injury (as described in U.S. Patent No. 5,494,896 and corresponding International Publication No. WO 96/30037 (PCT/US96/02349), both of which are incorporated herein by reference), ischemia/reperfusion injury (as described in co-owned, co-pending U.S. Application Serial No. 08/232,527 filed April 22, 1994, incorporated herein by reference), and liver resection (as described in co-owned, copending U.S. Application Serial No. 08/582,230 filed January 3, 1996, which is in turn a continuation of U.S. Application Serial No. 08/318,357 filed October 5, 1994, which is in turn a continuation-in-part of U.S. Application Serial No. 08/132,510 filed October 5, 1993, and corresponding International Publication No. WO 95/10297 (PCT/US94/11404), all of which are incorporated herein by reference).

BPI protein products are also known to neutralize the anticoagulant activity of exogenous heparin, as described in U.S. Patent No. 5,348,942, incorporated herein by reference, as well as to be useful for treating chronic inflammatory diseases such as rheumatoid and reactive arthritis and for inhibiting angiogenesis and for treating angiogenesis-associated disorders including malignant tumors, ocular retinopathy and endometriosis, as described in co-owned, co-pending U.S. Application Serial No. 08/415,158 filed March 31, 1995, which is in turn a continuation of U.S. Application Serial No. 08/093,202, filed July 15, 1993, which is in turn a continuation-in-part of U.S. Application Serial No. 08/030,644, filed March 12, 1993, all of which are incorporated herein by reference.

BPI protein products are also known for use in antithrombotic methods, as described in co-owned, co-pending U.S. Application Serial No. 08/644,290 filed May 10, 1996, incorporated herein by reference.

As used herein, "BPI protein product" includes naturally and recombinantly produced BPI protein; natural, synthetic, and recombinant biologically active polypeptide fragments of BPI protein; biologically active polypeptide variants of BPI protein or fragments thereof, including hybrid fusion proteins and dimers; biologically active polypeptide analogs of BPI protein or fragments or variants thereof, including cysteine-substituted analogs; and BPI-derived peptides. The BPI protein products administered according to this invention may be generated and/or isolated by any means known in the art. U.S. Patent No. 5,198,541, the disclosure of which is incorporated herein by reference, discloses recombinant genes encoding, and methods for expression of, BPI proteins including recombinant BPI holoprotein, referred to as rBPI and recombinant fragments of BPI. U.S. Patent No. 5,439,807 and corresponding International Publication No. WO 93/23540 (PCT/US93/04752), which are all incorporated herein by reference, disclose novel methods for the purification of recombinant BPI protein products expressed in and secreted from genetically transformed mammalian host cells in culture and discloses how one may produce large quantities of recombinant BPI products suitable for incorporation into stable, homogeneous pharmaceutical preparations.

Biologically active fragments of BPI (BPI fragments) include biologically active molecules that have the same or similar amino acid sequence as a natural human BPI holoprotein, except that the fragment molecule lacks amino-terminal amino acids, internal amino acids, and/or carboxy-terminal amino acids of the holoprotein. Nonlimiting examples of such fragments include an N-terminal fragment of natural human BPI of approximately 25 kD, described in Ooi et al., *J. Exp. Med., 174*:649 (1991), and the recombinant expression product of DNA encoding N-terminal amino acids from 1 to about 193 to 199 of natural human BPI, described in Gazzano-Santoro et al., *Infect. Immun. 60*:4754-4761 (1992), and referred to as rBPI₂₃. In that publication, an expression vector was used as a source of DNA encoding a recombinant expression product (rBPI₂₃) having the 31-residue signal sequence and the first 199 amino acids of the N-terminus of the mature human BPI, as set out in Figure 1 of Gray et al., *supra*, except that valine at position 151 is specified by GTG rather than GTC and residue 185 is glutamic acid (specified by GAG) rather than lysine (specified by AAG). Recombinant holoprotein (rBPI) has also been produced having the sequence (SEQ ID NOS: 145 and 146) set out in Figure 1 of Gray et al., *supra*, with the exceptions noted for rBPI₂₃ and with the exception that residue 417 is alanine (specified by GCT) rather than valine (specified by GTT). Other examples include dimeric forms of BPI fragments, as described in U.S. Patent No. 5,447,913 and corresponding International Publication No. WO 95/24209 (PCT/US95/03125), all of which are incorporated herein by reference.

Biologically active variants of BPI (BPI variants) include but are not limited to recombinant hybrid fusion proteins, comprising BPI holoprotein or biologically active fragment thereof and at least a portion of at least one other polypeptide, and dimeric forms of BPI variants. Examples of such hybrid fusion proteins and dimeric forms are described in co-owned, copending U.S. Application Serial No. 07/885,911 filed May 19, 1992, and a continuation-in-part application thereof, U.S. Application Serial No. 08/064,693 filed May 19, 1993 and corresponding International Publication No. WO 93/23434 (PCT/US93/04754), which are all incorporated herein by reference and include hybrid fusion proteins comprising, at the amino-terminal end, a BPI protein or a biologically active fragment thereof and, at the carboxy-terminal end, at least one constant domain of an immunoglobulin heavy chain or allelic variant thereof.

Biologically active analogs of BPI (BPI analogs) include but are not limited to BPI protein products wherein one or more amino acid residues have been replaced by a different amino acid. For example, U.S. Patent No. 5,420,019 and corresponding International Publication No. WO 94/18323 (PCT/US94/01235), all of which are incorporated herein by reference, discloses polypeptide analogs of BPI and BPI fragments wherein a cysteine residue is replaced by a different amino acid. A stable BPI protein product described by this application is the expression product of DNA encoding from amino acid 1 to approximately 193 or 199 of the N-terminal amino acids of BPI holoprotein, but wherein the cysteine at residue number 132 is substituted with alanine and is designated rBPI₂₁Δcys or rBPI₂₁. Production of this N-terminal analog of BPI, rBPI₂₁, has been described in Horwitz et al., *Protein Expression Purification, 8*:28-40 (1996). Other examples include dimeric forms of BPI analogs; e.g. U.S. Patent No. 5,447,913 and corresponding International Publication No. WO 95/24209 (PCT/US95/03125), all of which are incorporated herein by reference.

Other BPI protein products useful according to the methods of the invention are peptides derived from or based on BPI produced by recombinant or synthetic means (BPI-derived peptides), such as those described in International Publication No. WO 95/19372 (PCT/US94/10427), which corresponds to U.S. Application Serial No. 08/306,473, filed September 15, 1994, and International Publication No. WO94/20532 (PCT/US94/02465), which corresponds to U.S. Application Serial No. 08/209,762, filed March 11, 1994, which is a continuation-in-part of U.S. Application Serial No. 08/183,222, filed January 14, 1994, which is a continuation-in-part of U.S. Application Serial No. 08/093,202 filed July 15, 1993 (corresponding to International Publication No. WO 94/20128 (PCT/US94/02401)), which is a continuation-in-part of U.S. Application Ser. No. 08/030,644 filed March 12, 1993, the disclosures of all of which are incorporated herein by reference.

As used herein, an "N-terminal BPI protein product" as differentiated from a "BPI protein product" includes natural, synthetic, and recombinant biologically active N-terminal polypeptide fragments of BPI protein having a molecular weight of about 25 kd or less; biologically active polypeptide analogs of these N-terminal BPI fragments, including cysteine-substituted analogs; biologically active polypeptide variants comprising such N-terminal BPI fragments or analogs thereof, including hybrid fusion proteins and dimers; and peptides derived from or based on N-terminal BPI protein having a molecular weight of about 25 kd or less (BPI-derived peptides).

Presently preferred BPI protein products include recombinantly-produced N-terminal fragments of BPI, especially those having a molecular weight of approximately between 20 to 25 kD such as rBPI₂₁ or rBPI₂₃, or dimeric forms of these N-terminal fragments (e.g., rBPI₄₂ dimer). Preferred N-terminal dimeric products include dimeric BPI protein products wherein the monomers are N-terminal BPI fragments having the N-terminal residues from about 1 to 175 to about 1 to 199 of BPI holoprotein. A particularly preferred N-terminal dimeric product is the dimeric form of the BPI fragment having N-terminal residues 1 through 193, designated rBPI₄₂ dimer. Additionally, preferred N-terminal BPI protein products include rBPI and BPI-derived peptides.

The administration of N-terminal BPI protein products is preferably accomplished with a pharmaceutical composition comprising an N-terminal BPI protein product and a pharmaceutically acceptable diluent, adjuvant, or carrier. The N-terminal BPI protein product may be administered without or in conjunction with known surfactants, other chemotherapeutic agents or additional known anti-chlamydial agents. A stable pharmaceutical composition containing BPI protein products (e.g., rBPI₂₃) comprises the BPI protein product at a concentration of 1 mg/ml in citrate buffered saline (5 or 20 mM citrate, 150 mM NaCl, pH 5.0) comprising 0.1% by weight of poloxamer 188 (Pluronic F-68, BASF Wyandotte, Parsippany, NJ) and 0.002% by weight of polysorbate 80 (Tween 80, ICI Americas Inc., Wilmington, DE). Another stable pharmaceutical composition containing BPI protein products (e.g., rBPI₂₁) comprises the BPI protein product at a concentration of 2 mg/ml in 5 mM citrate, 150 mM NaCl, 0.2% poloxamer 188 and 0.002% polysorbate 80. Such preferred combinations are described in U.S. Patent No. 5,488,034 and corresponding International Publication No. WO 94/17819 (PCT/US94/01239), the disclosures of all of which are incorporated herein by reference. As described in U.S. Application Serial No. 08/586,133 filed January 12, 1996, which is in turn a continuation-in-part of U.S. Application Serial No. 08/530,599 filed September 19, 1995, which is in turn a continuation-in-part of U.S. Application Serial No. 08/372,104 filed January 13, 1995, and corresponding International Publication No. WO96/21436 (PCT/US96/01095), all of which are incorporated herein by reference, other poloxamer formulations of BPI protein products with enhanced activity may be utilized.

Therapeutic compositions comprising N-terminal BPI protein product may be administered systemically or topically. Systemic routes of administration include oral, intravenous, intramuscular or subcutaneous injection (including into a depot for long-term release), intraocular and retrobulbar, intrathecal, intraperitoneal (e.g. by intraperitoneal lavage), intrapulmonary (using powdered drug, or an aerosolized or nebulized drug solution), or transdermal.

When given parenterally, N-terminal BPI protein product compositions are generally injected in doses ranging from 1 µg/kg to 100 mg/kg per day, preferably at doses ranging from 0.1 mg/kg to 20 mg/kg per day, more preferably at doses ranging from 1 to 20 mg/kg/day and most preferably at doses ranging from 2 to 10 mg/kg/day. The treatment may continue by continuous infusion or intermittent injection or infusion, at the same, reduced or increased dose per day for, *e.g.*, 1 to 3 days, and additionally as determined by the treating physician. When administered intravenously, N-terminal BPI protein products are preferably administered by an initial brief infusion followed by a continuous infusion. The preferred intravenous regimen is a 1 to 20 mg/kg brief intravenous infusion of N-terminal BPI protein product followed by a continuous intravenous infusion at a dose of 1 to 20 mg/kg/day, continuing for up to one week. A particularly preferred intravenous dosing regimen is a 1 to 4 mg/kg initial brief intravenous infusion followed by a continuous intravenous infusion at a dose of 1 to 4 mg/kg/day, continuing for up to 72 hours.

Topical routes include administration in the form of salves, creams, jellies, ophthalmic drops or ointments (as described in co-owned, co-pending U.S. Application Serial No. 08/557,289 and 08/557,287, both filed November 14, 1995), ear drops, suppositories, irrigation fluids (for, e.g., irrigation of wounds) or medicated shampoos. For example, for topical administration in drop form, about 10 to 200 µL of an N-terminal BPI protein product composition may be applied one or more times per day as determined by the treating physician.

Intrapulmonary administration of N-terminal BPI protein products, alone or in addition to intravenous administration, is particularly preferred for the treatment of cystic fibrosis patients. Improved formulations of BPI protein products, especially N-terminal BPI protein products, that avoid precipitation of the nebulized drug are described herein. Such formulations are useful in methods of treating cystic fibrosis patients with BPI protein products.

Formulations contemplated by the invention comprise poloxamer surfactant at concentrations of 0.3% by weight or more, 0.4% by weight or more, 0.5% by weight or more, 0.6% by weight or more, 0.7% by weight or more, and 0.8% by weight or more, preferably at a range between about 0.3% and 3.0% by weight. Exemplary poloxamer surfactants include poloxamer 188 (available as PLURONIC F68, BASF, Parsippany, NJ), poloxamer 333 (available as PLURONIC P103, BASF) and poloxamer 403 (available as PLURONIC P123, BASF). Such formulations typically comprise 150 mM NaCI and may or may not include a buffer such as citrate, phosphate or MOPS, and optionally contain ethylenediaminetetraacetic acid (EDTA) at concentrations of, for example, 0.1% or 0.35% by weight, and also optionally contain a polysorbate (polyoxyethylene sorbitan fatty acid ester) surfactant such as polysorbate 80 (available as TWEEN 80, ICI Americas Inc., Wilmington, DE).

Those skilled in the art can readily optimize effective dosages and administration regimens for therapeutic compositions comprising N-terminal BPI protein product, as determined by good medical practice and the clinical condition of the individual patient. "Concurrent administration," or co-administration, as used herein includes administration of the agents, in conjunction or combination, together, or before or after each other. The N-terminal BPI protein product and second agent(s) may be administered by different routes. For example, the N-terminal BPI protein product may be administered intravenously while the second agent(s) is(are) administered intramuscularly, intravenously, subcutaneously, orally or intraperitoneally. Alternatively, the N-terminal BPI protein product may be administered in an aerosolized or nebulized form for intrapulmonary delivery, while the second agent(s) is(are) administered, e.g., intravenously. The N-terminal BPI protein product and second agent(s) may be given sequentially in the same intravenous line or nebulizer, or may be given in different intravenous lines or nebulizers. The formulated BPI protein product and second agent(s) may be administered simultaneously or sequentially, as long as they are given in a manner sufficient to allow all agents to achieve effective concentrations at the site of infection.

Other aspects and advantages of the present invention will be understood upon consideration of the following illustrative examples. Example 1 addresses the determination of BPI reactivity in the sera of cystic fibrosis patients. Example 2 addresses various formulations of BPI protein products for aerosol delivery.

### EXAMPLE 1

### Measurement of BPI Antibody Titers in Fluid Samples from Cystic Fibrosis Patients

Plasma samples from 11 cystic fibrosis (CF) patients were tested for the presence of anti-BPI antibodies as follows. BPI holoprotein (rBPI) or an N-terminal BPI protein product (rBPI₂₁) were used as antigen sources in an enzyme immunoassay. The wells of Immulon 2 microtiter plates (Dynatech Laboratories Inc., Chantilly, VA) were coated overnight at 2-8°C with 50 uL of rBPI₂₁ (0.5 µg/mL) or rBPI (1 µg/mL) diluted in phosphate buffered saline, pH 7.2 (PBS). Unbound BPI was removed and 200 µL of PBS containing 0.1% human serum albumin and 0.1% goat serum was added to all wells. After blocking the plates for 1 hour at room temperature, the wells were washed 3 times with 300 µL of wash buffer (PBS/0.5% Tween 20). Plasma samples were prepared as serial two-fold dilutions from 1/100 to 1/12,800. Plasma samples were diluted in triplicate with PBS containing 1 % bovine serum albumin, 1 % goat serum and 0.05 % Tween 20 (PBS-BSA-GS/Tween). The replicates and dilutions for each plasma sample were transferred (50 µL) to the treated microtiter plates and incubated for 1 hour at 37°C. After the primary incubation, the wells were washed 3 times with wash buffer. Alkaline phosphatase conjugated goat anti-human IgG, IgA and IgM (H+L) antibodies (Zymed Laboratories Inc., San Francisco, CA) were diluted 1/3000 in PBS-BSA-GS/Tween and 50 µL was added to all wells. The plates were then incubated for 1 hour at 37°C. Afterwards, all wells were washed 3 times with wash buffer and 3 times with deionized water. The substrate p-nitrophenylphosphate (1 mg/mL in 10% diethanolamine buffer, pH 9.8) was added in volume of 50 µL to all wells. Color development was allowed to proceed for 1 hour at room temperature, after which 50 µL of IN NaOH was added to stop the reaction. The absorbance at 405 nm (A₄₀₅) was determined for all wells using a Vmax Plate Reader (Molecular Devices Corp., Menlo Park, CA).

The mean A₄₀₅ for all samples were corrected for background by subtracting the mean A₄₀₅ of wells receiving sample dilution buffer (no plasma) in the primary incubation step. A linear-log plot of corrected mean A₄₀₅ versus the reciprocal dilution (titer) was constructed for each sample. An absorbance greater than 0.05 units was considered to be above background.

A detectable immune response (A₄₀₅ > 0.05) against rBPI was measured in 7/11 plasma samples, consistent with the 91% detection rate (61/66 samples) reported in Zhao, 1996, *supra*. Under the assay conditions described, no immune response was detected against rBPI₂₁ for any of the 11 CF plasma samples tested. For the CF plasma samples tested, immunoreactivity appears to be directed toward holo-BPI and not to the recombinant N-terminal BPI protein product, rBPI₂₁ (Neuprex™). This data suggests that anti-neutrophil cytoplasmic antibodies (ANCA) in CF patients are elicited only against the C-terminus of BPI. This restricted immune response to the C-terminus of BPI has been observed in plasma from other non-CF ANCA diseases (see Stoffel et al., *Clin. Exp. Immunol., 104*:54-59 (1996); and co-owned, co-pending U.S. Application Serial No. 08/742,985 filed November 1, 1996).

### EXAMPLE 2

### Aerosolization Studies with BPI Protein Product Formulations

The activity and integrity of various formulations of a BPI protein product were examined after aerosolization by a nebulizer (Baxter Misty Neb). For these experiments, a 1.4 mg/mL solution of rBPI₂₁ formulated in 5mM citrate, 150mM NaCl, pH 5.0, 0.002% polysorbate 80 (TWEEN 80, ICI Americas Inc., Wilmington, DE), with concentrations of poloxamer 188 (PLURONIC F68, BASF, Parsippany, NJ) varying from 0% to 0.4% by weight, was used for nebulization. A liquid impinger was set up to collect the rBPI₂₁ aerosol droplets generated, and observations of visible precipitation were recorded as shown below in Table 1.

**Table 1**

| Sample | poloxamer 188 Conc. (% by weight) | polysorbate 80 Conc. (% by weight) | Visible Precipitate |
|---|---|---|---|
| 1 | 0.0 | 0.002 | yes |
| 2 | 0.0 | 0.002 | yes |
| 3 | 0.1 | 0.002 | yes |
| 4 | 0.1 | 0.002 | yes |
| 5 | 0.2 | 0.002 | yes |
| 6 | 0.2 | 0.002 | yes |
| 7 | 0.3 | 0.002 | no |
| 8 | 0.3 | 0.002 | no |
| 9 | 0.4 | 0.002 | no |
| 10 | 0.4 | 0.002 | no |

Extensive precipitation of rBPI₂₁ occurred in the nebulizer in the absence of poloxamer 188. Poloxamer 188 concentrations greater than 0.2% were necessary to prevent precipitation in a 1.4 mg/mL rBPI₂₁ solution in 5mM citrate, 150mM NaCl, pH 5.0, 0.002% polysorbate 80 (PS80). These data suggest that in the presence of 0.002% polysorbate 80, concentrations of greater than 0.2% poloxamer 188 are needed to prevent precipitation in the nebulizer for a 1.4 mg/mL rBPI₂₁ solution.

Nebulization time (time at which there was no visible aerosol exiting the nebulizer) was inversely proportional to the nebulizer airflow rate but had no effect on nebulizer efficiency (% of rBPI₂₁ which exits the nebulizer). A nebulizer efficiency of 75-79% for rBPI₂₁ formulated with 0.4% poloxamer 188 was observed at the three air flow rates tested (6 L/min, 8 L/min, and 10 L/min).

rBPI₂₁ formulated in 0.4% poloxamer 188 which was collected in the liquid impinger after nebulization showed no change in activity as determined by an LAL inhibition assay, no shift in retention time on an HPLC assay, and no difference on SDS-PAGE when compared to rBPI₂₁ before the nebulization process. Furthermore, rBPI₂₁ which remained in the nebulizer after the nebulization process was also just as active and showed no sign of structural changes by HPLC or SDS-PAGE.

A BPI protein product, rBPI₂₁, was further evaluated in formulations containing 150 mM NaCl, 0.35% EDTA, pH 6.0, with concentrations of poloxamer 333 (PLURONIC P103, BASF) varying from 0 to 0.8% by weight, with or without 0.002% by weight polysorbate 80 (TWEEN 80, ICI Americas) and with or without 2.5 mM citrate. The concentration of rBPI₂₁ in the formulations ranged from 1.4 to 1.9 mg/mL as indicated in Table 2 below. The visual appearance of these formulations after nebulization at an air flow rate of 10 L/min. was determined. The visual examination included a qualitative assessment of the amount of precipitate, which was represented by a numerical score from 0-4, with 0 representing a sample containing no precipitate and 4 representing the greatest amount of precipitate. Results of these studies are shown in Table 2 below.

**Table 2**

| Run # | Poloxamer 333 conc. (%) | 2.5 mM citrate | 0.002% polysorbate 80 | Volume (ml)^{a} | Time^{b} (min) | mg/mL rBPI₂₁ conc. start/ end^{c} | Visual score^{d} |
|---|---|---|---|---|---|---|---|
| 1 | 0 | yes | no | 2 | 8.5 | 1.9 | 4 |
| 2 | 0.1 | yes | no | 2 | 8.9 | 1.8 | 3 |
| 3 | 0.2 | yes | no | 2 | 7.7 | 1.7 | 2 |
| 4 | 0.4 | yes | no | 2 | 9.0 | 1.6 | 2 |
| 5 | 0.6 | yes | no | 2 | 8.8 | 1.5 | 1 |
| 6 | 0.8 | yes | no | 2 | 8.2 | 1.4/2.0 | 0 |
| 7 | 0.8 | yes | no | 2 | 8.0 | 1.7/2.0 | 0 |
| 8 | 0.2 | yes | yes | 2 | 8.0 | 1.8 | 2 |
| 9 | 0.4 | yes | yes | 2 | 8.7 | 1.6/2.2 | 0 |
| 10 | 0.4 | yes | yes | 2 | 8.0 | 1.7/2.4 | 0 |
| 11 | 0.4 | yes | yes | 2 | 7.7 | 1.7/2.1 | 0 |
| 12 | 0.4 | no | yes | 2 | 7.6 | 1.9 | 2 |
| 13 | 0.8 | no | no | 2 | 7.5 | 1.7/2.2 | 0 |
| 14 | 0.8 | yes | no | 5 | 38.0 | 1.5/3.2 | 0 |
| 15 | 0.4 | no | yes | 5 | 39.7 | 1.9 | 2 |
| 16 | 0.8 | no | no | 5 | 34.0 | 1.9/3.6 | 0 |

| | | | | | | | |
|---|---|---|---|---|---|---|---|
| ^{a} Volume in nebulizer reservoir before aerosolization. | | | | | | | |
| ^{b} Time until aerosol formation ceased. | | | | | | | |
| ^{c} The first value indicated is the BPI concentration in the nebulizer before aerosolization. A second value, if present, indicates BPI concentration in the nebulizer after aerosolization. | | | | | | | |
| ^{d} Visual scoring was as follows: 0 = clear, no pellet when centrifuged; 1 = clear, small pellet when centrifuged; 2 = precipitate visible; 3 = cloudy; and 4 = very cloudy. | | | | | | | |

The results shown in Table 2 above demonstrate that a formulation of rBPI₂₁ containing 0.8% poloxamer 333 prevented precipitation of rBPI₂₁ during aerosolization for at least 38 minutes. A formulation of rBPI₂₁ containing 0.4% poloxamer 333 and 0.002% polysorbate 80 prevented precipitation of rBPI₂₁ during aerosolization over short time frames (7.5 to 9 minutes), although precipitation was observed during longer aerosolization times.

Aerosol from two rBPI₂₁ formulations [(1) 2.5 mM citrate, 150 mM NaCl, pH 6.0, 0.35% EDTA, 0.8% poloxamer 333, or (2) 2.5 mM citrate, 150 mM NaCl, pH 6.0, 0.35% EDTA, 0.4% poloxamer 333, 0.002% polysorbate 80] was collected and analyzed for antimicrobial activity against *Pseudomonas aeruginosa* as follows. *P. aeruginosa* (ATCC No. 27853) was cultured for 24 hours on Trypticase Soy Agar (TSA) and diluted in deionized water. A 100 µL aliquot was inoculated into 25 mL of cation-supplemented Mueller-Hinton broth, the sample was mixed, and 450 µL aliquots were added to 50 µL of either saline, formulation buffer (containing no BPI) or formulated rBPI₂₁, to produce a final concentration of 20 µg/mL or 80 µg/mL rBPI₂₁. Samples were incubated at 37°C. After 1, 3, 5 and 24 hours of incubation, 10 µL samples were diluted in sterile water for injection and plated on TSA. After 48 hours of incubation at 37°C, colonies were counted.

The bioactivity of the collected aerosol was compared to that of the starting material before aerosolization and to that of the solution remaining in the nebulizer after aerosolization. Results show that for the rBPI₂₁ formulation containing 0.8% poloxamer 333, all of the samples were active and reduced CFU/ml to below detection within 3 hours. For the formulation containing 0.4% poloxamer 333 with 0.002% polysorbate 80, all samples were active and reduced CFU/ml to below detection within 3 hours, except that one of the collected aerosol samples reduced CFU/ml to below detection only at the 1 hour time point. Two control samples of formulation buffer alone (without rBPI₂₁) and growth media alone exhibited no antimicrobial activity.

A BPI protein product, rBPI₂₁, was also evaluated in the following formulations: (A) 5 mM citrate, 150 mM NaCl, pH 5.0, 0.2% poloxamer 188, 0.002% polysorbate 80; (B) 5 mM phosphate, 150 mM NaCl, pH 6.0; (C) 5 mM phosphate, 150 mM NaCl, pH 6.0, with 0.1% poloxamer 333; (D) 5 mM phosphate, 150 mM NaCl, pH 6.0, with 0.2% poloxamer 333; (E) 5 mM phosphate, 150 mM NaCl, pH 6.0, with 0.4% poloxamer 333; (F) 5 mM phosphate, 150 mM NaCl, pH 6.0, with 0.8% poloxamer 333; (G) 0.35% EDTA with 0.8% poloxamer 333. These protein solutions were nebulized, and the light scattering of the collected aerosols was determined by measuring absorbance at 320 nm. The results demonstrated that formulations E, F and G had little or no precipitate as detected by light scattering.

Certain results of related experiments are believed to be of interest to the present invention.

Analysis of broncheoalveolar lavage (BAL) fluids of 29 CF patients for the presence of endogenous BPI by the assay described in U.S. Patent Nos. 5,466,580 and 5,466,581, both of which are incorporated herein by reference, revealed the presence of elevated levels of BPI in 27 samples in comparison to 8 non-disease control BAL samples.

A *Pseudomonas aeruginosa* strain isolated from a sputum sample from a CF patient was found to be resistant to most of the antibiotics included in commercially available Dade MicroScan gram-negative panels. However, when tested in the same system supplemented with 16 µg/mL rBPI₂₁ (formulated as described above with 0.2% poloxamer 188), the organism appeared to be susceptible to combinations of rBPI₂₁ and aztreonam, carbenicillin, ciprofloxacin, imipenem, tobramycin and sulfamethoxazole/trimethoprim.

In combination with the same antibiotics, rBPI₂₁ formulated with 0.2% poloxamer 403 (PLURONIC P123®, BASF), rather than the usual 0.2% poloxamer 188, appeared to have even greater activity than the poloxamer 188 formulation. rBPI₂₁ in this formulation, in combination with antibiotic, further decreased the MICs for aztreonam, ciprofloxacin and imipenem and provided increased susceptibity to netilmicin, chloramphenicol, azlocillin and Augmentin.

The effects of different poloxamer formulations on the activities of BPI protein products are described in U.S. Application Serial No. 08/586,133 filed January 12, 1996, which is in turn a continuation-in-part of U.S. Application Serial No. 08/530,599 filed September 19, 1995, which is in turn a continuation-in-part of U.S. Application Serial No. 08/372,104 filed January 13, 1995, and corresponding International Publication No. WO96/21436 (PCT/US96/01095), all of which are incorporated herein by reference.

When the same *P. aeruginosa* strain was incubated with rBPI₂₁ alone (without antibiotic), the rBPI₂₁ formulated with poloxamer 403, which resulted in an MIC of < 16 µg/mL at 24 hours, was also found to be more active than the rBPI₂₁ formulated with poloxamer 188.

Preferred features of the invention are included, as follows:
1. In a method of treating a cystic fibrosis patient with a bactericidal/permeability-increasing (BPI) protein product, the improvement comprising administering an N-terminal BPI protein product to a subject having non-N-terminal-BPI-reactive antibodies.
2. The method of feature 1 wherein the N-terminal BPI protein product is being administered to the cystic fibrosis patient to ameliorate adverse effects associated with endotoxin in circulation.
3. The method of feature 1 wherein the N-terminal BPI protein product is being administered to the cystic fibrosis patient to ameliorate adverse effects associated with meningococcemia.
4. The method of feature 1 wherein the N-terminal BPI protein product is being administered to the cystic fibrosis patient to ameliorate adverse effects associated with hemorrhagic trauma.
5. The method of feature 1 wherein the N-terminal BPI protein product is being administered to the cystic fibrosis patient to ameliorate adverse effects associated with burn injury.
6. The method of feature 1 wherein the cystic fibrosis patient being treated is suffering from a gram-negative bacterial infection.
7. The method of feature 1 wherein the cystic fibrosis patient being treated is suffering from a gram-positive bacterial or mycoplasmal infection.
8. The method of feature 1 wherein the cystic fibrosis patient being treated is suffering from a fungal infection.
9. The method of feature 1 wherein the cystic fibrosis patient being treated is suffering from a protozoan infection.
10. The method of feature 1 wherein the cystic fibrosis patient being treated is suffering from a chlamydial infection.
11. The method of feature 1 wherein the cystic fibrosis patient being treated is suffering from a mycobacterial infection.
12. The method of feature 1 wherein the cystic fibrosis patient being treated is suffering from a chronic inflammatory disease.
13. The method of feature 1 wherein the N-terminal BPI protein product is being administered to cystic fibrosis patient to inhibit angiogenesis.
14. The method of feature 1 wherein the N-terminal BPI protein product is being administered to the cystic fibrosis patient to promote fibrinolysis.
15. The method of feature 1 wherein the N-terminal BPI protein product is an amino-terminal fragment of BPI protein having a molecular weight of about 20 kD to 25 kD.
16. The method of feature 1 wherein the N-terminal BPI protein product is rBPI₂₃ or a dimeric form thereof.
17. The method of feature 1 wherein the N-terminal BPI protein product is rBPI₂₁.
18. A composition for aerosol delivery comprising a BPI protein product and a poloxamer surfactant at a concentration of 0.3% or more.
19. A composition of feature 18 further comprising 0.002% or more polysorbate 80 by weight.
20. The composition of feature 18 wherein the poloxamer surfactant is poloxamer 188 at a concentration of 0.3% by weight.
21. The composition of feature 18 wherein the poloxamer surfactant is poloxamer 333 at a concentration of 0.4% by weight.
22. An improved method of administering a BPI protein product to a patient suffering from cystic fibrosis, comprising administering the composition of Claim 18 to said patient via aerosol delivery.

Numerous modifications and variations of the above-described invention are expected to occur to those of skill in the art. Accordingly, only such limitations as appear in the appended claims should be placed thereon.

## Claims

1. A composition for aerosol delivery comprising a bactericidal/permeability-increasing (BPI) protein product and a poloxamer surfactant, wherein the poloxamer surfactant is at a concentration of 0.3% by weight or more.

2. A composition according to Claim 1, wherein the poloxamer surfactant is at a concentration of 0.4% by weight or more, 0.5% by weight or more, 0.6% by weight or more, 0.7% by weight, or more or 0.8% by weight or more.

3. A composition according to Claim 1, wherein the poloxamer surfactant is at a concentration of between 0.3% and 3% by weight.

4. A composition according to any one of Claims 1 to 3, wherein the poloxamer surfactant is poloxamer 188, poloxamer 333 or poloxamer 403.

5. A composition according to Claim 1, wherein the poloxamer surfactant is poloxamer 188 at a concentration of 0.3% by weight.

6. A composition according to Claim 1, wherein the poloxamer surfactant is poloxamer 333 at a concentration of 0.4% by weight.

7. A composition according to any one of the preceding claims further comprising 0.002% or more polysorbate 80 by weight.

8. A composition according to any one of the preceding claims, further comprising ethylenediaminetetraacetic acid (EDTA) at a concentration of 0.1% or 0.35% by weight.

9. A composition according to any one of the preceding claims, wherein the BPI protein product is an N-terminal fragment of BPI protein having a molecular weight of from 20 kD to 25 kD.

10. A composition according to any one of Claims 1 to 8, wherein the BPI protein product is rBPI₂₃ or a dimeric form thereof.

11. A composition according to any one of Claims 1 to 8, wherein the BPI protein product is rBPI₂₁.

12. A composition according to any one of the preceding claims, further comprising another therapeutic agent.

13. A composition according to Claim 12, wherein the other therapeutic agent is an antimicrobial agent, including antibiotics and anti-fungal agents, DNAase (Pulmozyme®), a chemotherapeutic agent or an anti-chlamydial agent.

14. A composition according to any one of the preceding claims for treatment of a gram-negative bacterial infection, a gram-positive bacterial infection, a mycoplasmal infection, a fungal infection, a protozoan infection, a chlamydial infection, or a mycobacterial infection, for enhancing the effectiveness of an antimicrobial agent, for ameliorating adverse effects associated with endotoxin in circulation, meningococcemia, hemorrhagic trauma, burn injury, ischemia/reperfusion injury, or liver resection injury, for treatment of chronic inflammatory diseases, including rheumatoid and reactive arthritis, for inhibiting angiogenesis, for treating angiogenesis-associated disorders including malignant tumors, ocular retinopathy and endometriosis, for neutralising anticoagulant activity of exogenous heparin, for promoting fibrinolysis, or for use in antithrombotic methods.

15. A composition according to any one of Claims 9 to 11 for use in treating a cystic fibrosis patient.

16. A composition according to any one of the preceding claims for intrapulmonary administration.

17. An aerosol comprising a composition according to any one of the preceding claims.

18. An aerosol according to Claim 17, wherein the aerosol is a nebulizer or a metered dose inhaler.

19. A jet nebulizer according to Claim 18.

20. An ultrasonic nebulizer according to Claim 18.

21. Use of a composition according to any one of Claims 1 to 13 for the manufacture of a medicament for treatment of a gram-negative bacterial infection, a gram-positive bacterial infection, a mycoplasmal infection, a fungal infection, a protozoan infection, a chlamydial infection, or a mycobacterial infection, for enhancing the effectiveness of an antimicrobial agent, for ameliorating adverse effects associated with endotoxin in circulation, meningococcemia, hemorrhagic trauma, burn injury, ischemia/reperfusion injury, or liver resection injury, for treatment of chronic inflammatory diseases, including rheumatoid and reactive arthritis, for inhibiting angiogenesis, for treating angiogenesis-associated disorders including malignant tumors, ocular retinopathy and endometriosis, for neutralising anticoagulant activity of exogenous heparin, for promoting fibrinolysis, or for use in antithrombotic methods.

22. Use of a composition of any one of Claims 9 to 11 for the manufacture of a medicament for treatment of a cystic fibrosis patient.

23. A use according to Claim 21 or 22, when dependent on any one of Claims 1 to 11, for concurrent administration with another therapeutic agent.

24. A use according to Claim 23, wherein the other therapeutic agent is an antimicrobial agent, including antibiotics and anti-fungal agents, DNAase (Pulmozyme®), a chemotherapeutic agent or an anti-chlamydial agent.

25. A use according to anyone of Claims 21 to 24 wherein the medicament is for intrapulmonary administration.

26. An aerosol which comprises a composition comprising a BPI protein product and a poloxamer surfactant, wherein the poloxamer surfactant is present at a concentration effective to avoid precipitation of the BPI protein product during aerosol delivery.

27. An aerosol according to Claim 26, wherein said aerosol is a nebulizer or a metered dose inhaler.

28. Use of a BPI protein product composition comprising a BPI protein product and a poloxamer surfactant for the manufacture of a medicament for treatment of a cystic fibrosis patient by aerosol delivery, wherein said poloxamer surfactant is present at a concentration effective to avoid precipitation of the BPI protein product during aerosol delivery.
